# EUROPEAN PATENT APPLICATION

(11) **EP 2 924 040 A1**
(43) Date of publication of application: **30.09.2015**
(21) Application number: 14162148.2
(22) Date of filing: 27.03.2014
(51) Int. Cl.: C07D 487/04, A61K 31/497, A61P 35/00

(54) **2-Amino-1-(o-tolyl)pyrrolo[3,2-b]quinoxaline-3-carboxamide derivates**

(71) Applicant: Universität Zürich, 8006 Zürich (CH)
(72) Inventor: Zhao, Hongtao, CH-8044 Zurich (CH); Huang, Danzhi, 8050 Zurich (CH); Lafleur, Karine Anne, 4310 Rheinfelden (CH); Unzue Lopez, Andrea, 8050 Zurich (CH); Frugier, Emilie, 5400 Baden (CH); Nevado Blazquez, Cristina, 8057 Zurich (CH); Caflisch, Amedeo, 8057 Zurich (CH)
(74) Representative: Bolsinger, Jens Roland

(57) **Abstract**

The invention relates to a compound characterized by a general formula (1), 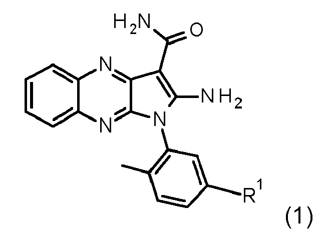 wherein R¹ is OH or CH₂OH. The invention relates further to a pharmaceutical preparation comprising at least one of said compounds. A further aspect of the invention the use of said compound or said pharmaceutical preparation in a method of treatment of disease, in particular the treatment of cancer or intraocular neovascular syndromes.

## Description

### Field of the invention

The invention relates to 2-amino-1-(o-tolyl)pyrrolo[3,2-b]quinoxaline-3-carboxamide derivates, pharmaceutical preparations thereof and their use in the treatment of diseases, in particular of cancer and intraocular neovascular syndromes.

### Background of the invention

Receptor tyrosine kinases are attractive targets for cancer therapeutics. For instance, the receptor tyrosine kinase EphB4 is involved in many types of cancer (Cheng N., Brantley D.M. and Chen J., Cytokine Growth Factor Rev., 2002, 13:75-85).

Ephrins and their receptors were first identified in studies for neuronal growth during development. Unlike other families of receptor tyrosine kinases, which bind soluble ligands, ephrin receptors interact with cell surface-bound ephrin ligands. Ephrins attach to the cell membrane either through a glycosylphosphatidyl inositol anchor (ephrin A) or a transmembrane domain (ephrin B). Ephrin receptors are likewise divided in two subclasses EphA and EphB, depending on the type of interaction with their ligands ephrin A or B. Ephrins and their receptors have been shown to play an essential role in vascular development during embryogenesis and in adult angiogenesis, as key regulators of vascular assembly, arteriovenous differentiation, and boundary formation. Both EphA and EphB receptors and their ligands are involved in vascular development. Especially, ephrin B2 is expressed in arterial endothelial cells, whereas its cognate receptor, EphB4 is expressed in venous endothelial cells. EphB4 (also named HTK) and its ligand, ephrin B2 (HTKL), play important roles in establishing and determining vascular networks. Blocking this receptor/ligand pair inhibits the end stage of tumour blood vessel formation, as well as causes direct growth inhibition of certain cancers.

In this context, inhibitors that bind to their target receptor with high selectivity as compared to other targets are often highly desirable to limit off-target binding, which is unproductive and can lead to undesired side-effects.

EphB4 seems to be rather recalcitrant to inhibition. Despite its potential therapeutic importance, few non-peptidic small molecule inhibitors have been reported in the literature up to date (Miyazaki Y. et al., Bioorg. Med. Chem. Lett. 2007, 17:250-254, Zhao H. and Huang D., PLoS ONE, 2011, Volume 6, Issue 6; Martiny-Baron G. et al., Angiogenesis, 2010, Volume 13, Issue 3: 259-67).

The 2012 doctoral thesis of Lafleur "Design and Synthesis of Selective Kinase Inhibitors" (available at the Zentralbibliothek Zürich, in Zurich, Switzerland) describes the lead optimization and fluorescence properties of two EphB4 Inhibitors, namely 2-amino-1-(3-methoxyphenyl)pyrrolo[3,2-b]quinoxaline-3-carboxamide and 2-amino-1-(3-fluorophenyl) pyrrolo[3,2-b]quinoxaline-3-carboxamide. Concerning the lead optimization, Lafleur refers to different possible substitutions on the phenyl ring (see formula I) in order to achieve different effects. For example, in order to increase the van der Waals interactions with side chains of Val626, Ala645, and Thr693, alkyl chains are applied as substituents (page 126, first paragraph). Formation of additional hydrogen bonds between an inhibitor and the side chain of Glu664 by providing a hydrogen bond donor on the phenyl ring is also discussed (page 126, second paragraph). Furthermore, the effect of an increase in steric hindrance and a disruption of molecular planarity in terms of selectivity and solubility is discussed (page 126, third paragraph).

It has further to be noted that Lafleur describes rather undefined compounds of the general formula (I) 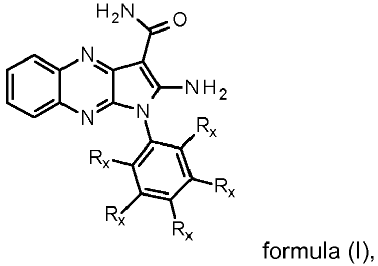 wherein an unambiguous definition of the substituents Rₓ is not given (see page 127; scheme 2). Furthermore, no clear indication with respect to a certain substitution pattern is given. This is due to the fact that there is no differentiation between the individual substituents Rₓ on the phenyl ring, since each substituent is simply referred to as Rₓ.

Even if the examples of table 2 (page 128) are taken into consideration, it is not clear without ambiguity which chemical groups fall under the definition of Rₓ. It appears that one Rₓ of the five non-distinguished Rₓ may comprise an alkyl group. Another one Rₓ of the five non-distinguished Rₓ may comprise an alkyl group or a halogen. A further one Rₓ of the five non-distinguished Rₓ may comprise a hydrogen bond acceptor or a hydrogen donor. With the exception of one example (see line two, page 128, concerning hydrogen bond donors) no definition is given as to which chemical groups fall under the definition of hydrogen bond acceptors or hydrogen bond donors. Thus, any chemical group which can provide or accept hydrogen bonds will fall under this definition. Furthermore, there is also no concluding definition of alkyl, such that any possible alkyl group will fall under this definition. Given the lack of examples or clear definitions, the general formula (I) is unclear with respect to the described possible substituents Rₓ. At best, the general formula (I) can be seen as a generic formula with rather broad "lists" (two or more) of substituents for each of the five non-distinguished substituents Rₓ. Furthermore, considering the lack of differentiation between the individual substituents Rₓ on the phenyl ring, a clear substitution pattern on the phenyl ring is not unambiguously disclosed in the thesis.

The thesis discloses only unspecific compounds due to the unclear or undefined substituents Rₓ on the phenyl ring. Furthermore, the use of a donor-acceptor moiety or the provision of compounds comprising a high selectivity to protein kinases is not disclosed.

The objective of the invention is to provide 2-amino-1-(o-tolyl)pyrrolo[3,2-b]quinoxaline-3-carboxamide derivates which comprise a donor-acceptor moiety. These compounds particularly comprise a high selectivity to protein kinases, and notably to one or more protein-tyrosine kinases. Further objects of the inventions are to provide pharmaceutical preparations thereof and the use of the compounds or pharmaceutical preparations in the treatment of diseases, in particular of cancer and intraocular neovascular syndromes.

This objective is attained by the subject-matter of the independent claims.

### Summary of the invention

An aspect of the invention relates to a compound characterized by a general formula 1, 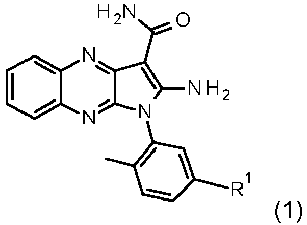 wherein R¹ is OH or CH₂OH.

Another aspect of the invention relates to a pharmaceutical preparation for treatment of diseases, in particular of cancer or intraocular neovascular syndromes comprising at least one compound of the general formula 1.

A further aspect of the invention relates to a compound or a pharmaceutical preparation according to the invention for use in a method of treatment of disease.

Another aspect of the invention relates to a compound or a pharmaceutical preparation according to the invention for use in a method for treatment of cancer or intraocular neovascular syndromes, in particular for use in a method for treatment of cancer.

As used herein the term "parent molecule," refers to the 2-amino-1-phenyl-pyrrolo[3,2-b]quinoxaline-3-carboxamide basis structure 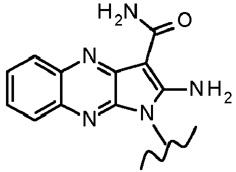 of the compounds of the invention.

As used herein the term "Donor-Acceptor" refers to a moiety, which is able to provide a hydrogen bonding as a hydrogen donor and a hydrogen bonding as a hydrogen acceptor to an adjacent molecule.

### Detailed description of the invention

According to one aspect the invention relates to compounds of the a general formula (1), 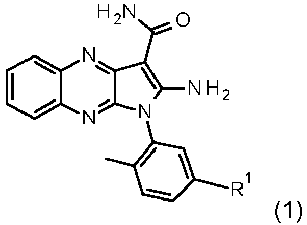 wherein R¹ is OH or CH₂OH .

The substituent R¹ is in meta position in relation to the attachment position of the benzene moiety to the parent moiety and in para position to the attachment position of the CH₃-moety yielding a 1, 2, 5 substitution pattern with the parent moiety being in the position 1. This substitution pattern favorably distorts the planarity of the inventive compounds and increases their solubility in aqueous media, while optimizing the inventive compound conformation within and volume-filling of the target protein binding site.

Furthermore, the substituent R¹ comprises a Donor-Acceptor moiety, namely a functional group OH, which is capable of providing hydrogen bonds as well as accepting hydrogen bonds, notably to Glu664 of EphB4.

In some embodiments, the compound of the invention is 2-amino-1-(5-hydroxy-2-methyl-phenyl)pyrrolo[3,2-b]quinoxaline-3-carboxamide 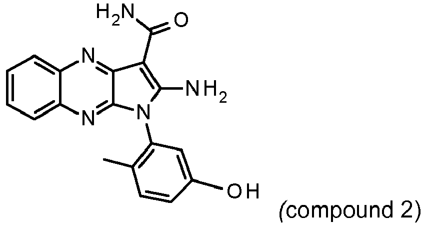 In some embodiments, the compound of the invention is 2-amino-1-[5-(hydroxymethyl)-2-methyl-phenyl]pyrrolo[3,2-b]quinoxaline-3-carboxamide

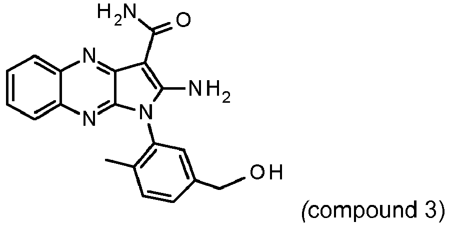

In some embodiments, the compounds of the general formula 1 may be isolated in form of salts, in particular in form of pharmaceutically acceptable salts. The same applies to all of the before mentioned embodiments.

In some embodiments, the compounds of the general formula 1 may be isolated in form of a tautomer, a hydrate or a solvate.

Such salts are formed, for example, as acid addition salts, preferably with organic or inorganic acids, from compounds of the general formula 1 with a basic nitrogen atom, in particular the pharmaceutically acceptable salts are formed in such a way. Suitable inorganic acids are, without being limited to, halogen acids, such as hydrochloric acid, sulfuric acid, or phosphoric acid and the like. Suitable organic acids are, without being limited to, carboxylic, phosphonic, sulfonic or sulfamic acids and the like. Such organic acids may be, without being limited to, acetic acid, glycolic acid, lactic acid, malic acid, tartaric acid, or citric acid.

Salts may also be formed, for example, as salts with organic or inorganic bases, from compounds of the general formula 1 with a nitrogen atom bearing an acidic hydrogen. Examples of suitable cations are - without being limited to - sodium, potassium, calcium or magnesium cations, or cations of organic nitrogen bases, e.g. protonated mono-, di- or tri-(2-hydroxethyl)amine.

In view of the close relationship between the novel compounds in their free form and those in the form of their salts, any reference to the free compounds hereinbefore and hereinafter is to be understood as referring also to the corresponding salts, as appropriate and expedient. Likewise, in view of the close relationship between the novel compounds of the general formula 1 and their tautomers, any reference to the compounds of the general formula 1 is to be understood as referring also to the corresponding tautomers. The same applies to a hydrate or a solvate.

Another aspect of the invention relates to a pharmaceutical preparation comprising at least one compound according to one of the claims 1 to 3 as an active ingredient.

In some embodiments, the pharmaceutical preparation comprises at least one compound according to the invention as an active ingredient and at least one pharmaceutically acceptable carrier.

In some embodiments, the pharmaceutical preparation comprises at least one compound according to the invention in its free form as an active ingredient.

In some embodiments, the pharmaceutical preparation comprises at least one compound according to the invention in its free form as an active ingredient and at least one pharmaceutically acceptable carrier.

In some embodiments, the pharmaceutical preparation comprises at least one compound according to the invention in form of a salt, a tautomer, a pharmaceutically acceptable salt, a hydrate or a solvate.

In some embodiments, the pharmaceutical preparation comprises at least one compound according to the invention in form of a salt, a tautomer, a pharmaceutically acceptable salt, a hydrate or a solvate and at least one pharmaceutically acceptable carrier.

Furthermore the invention relates to pharmaceutical preparations comprising at least one compound mentioned hereinbefore as active ingredient, which can be used especially in the treatment of the diseases mentioned. The pharmaceutical preparations may be used in particular for a method for treatment of cancer or intraocular neovascular syndromes, more particularly for a method for treatment of cancer.

In some embodiments, the pharmaceutical preparations is for enteral administration, such as nasal, buccal, rectal or, especially, oral administration, and for parenteral administration, such as intravenous, intramuscular or subcutaneous administration, to warm-blooded animals, in particular humans, are especially preferred. The preparations comprise the active ingredient alone or, in particular, together with a pharmaceutically acceptable carrier. The dosage of the active ingredient depends upon the disease to be treated and upon the species, its age, weight, and individual condition, the individual pharmacokinetic data, and the mode of administration.

In some embodiments, a pharmaceutical preparation for the treatment of angiogenesis dependent cancers and intraocular neovascular syndromes, in particular for a method for treatment of cancer, of a warm-blooded animal, in particular a human requiring such treatment, comprises a compound of the general formula 1 as active ingredient in a quantity that is therapeutically active against the said diseases.

In some embodiments, the pharmaceutical preparations comprise from approximately 1% to approximately 95% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

In some embodiments, the pharmaceutical preparations of the present invention are prepared in a manner known per se, for example by means of conventional mixing, granulating, coating, dissolving or lyophilizing processes.

In some embodiments, the pharmaceutical preparations is in form of solutions of the active ingredient, and also suspensions or dispersions, especially isotonic aqueous solutions, dispersions or suspensions which, for example in the case of lyophilized preparations comprising the active ingredient alone or together with a carrier, for example mannitol, can be made up before use.

In some embodiments, the pharmaceutical preparations may be sterilized and/or may comprise excipients, for example preservatives, stabilizers, wetting agents and/or emulsifiers, solubilizers, salts for regulating osmotic pressure and/or buffers and are prepared in a manner known per se, for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80® (polyoxyethylene(20)sorbitan mono-oleate).

In some embodiments, the pharmaceutical preparation comprises suspensions in oil, which comprise as the oil component a vegetable, synthetic, or semi-synthetic oils customary for injection purposes.

In some embodiments, the pharmaceutical preparation comprises liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol.

In some embodiments, the pharmaceutical preparation comprises mixtures of fatty acid esters, vegetable oils such as, without being limited to, cottonseed oil, almond oil, olive oil, castor oil, sesame oil, soybean oil and groundnut oil.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinyl pyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or tablet coatings, for example for identification purposes or to indicate different doses of active ingredient.

In some embodiments, the pharmaceutical preparation is suitable for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxy ethylene sorbitan fatty acid ester type, may also be added.

In some embodiments, the pharmaceutical preparation is suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

In some embodiments, the pharmaceutical preparation is suitable for parenteral administration, aqueous solutions of an active ingredient in water-soluble form or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents. Solutions such as are used, for example, for parenteral administration can also be employed as infusion solutions.

Preferred preservatives are, for example, antioxidants, such as ascorbic acid, or microbicides, such as sorbic acid or benzoic acid.

A further aspect of the invention relates to a compound according to any of the claims 1 to 3 or a pharmaceutical preparation according to claim 4 for use in a method of treatment of disease.

The compounds of the general formula 1 have valuable pharmacological properties.

Compounds of the general formula 1 inhibit Ephrin receptor kinases, in particular EphB4 kinase, are modulating angiogenesis, and are especially appropriate for the use against diseases or disorders such as cancer including, but not limited to, colon cancer and angiogenesis-dependent cancers, intraocular neovascular syndromes and related diseases, e.g. psoriasis and rheumathoid arthritis (see e.g. Folkman et al., J. Biol. Chem. 267:10931-34 (1992); Klagsbrun et al., Annu. Rev. Physiol. 53:217-39 (1991); and Garner A., In: Pathobiology of Ocular Disease. A Dynamic Approach, 2nd Edition (Marcel Dekker, NY, 1994), pp 1625-1710).

Angiogenesis-dependent cancers are, for example, so-called solid tumors, especially cancers of the gastrointestinal tract, the pancreas, breast, stomach, cervix, bladder, kidney, prostate, ovaries, endometrium, lung, brain, melanoma, Kaposi's sarcoma, squamous cell carcinoma of head and neck, malignant pleural mesotheriorna, lymphoma or multiple myeloma, also haemangioblastoma and haemangioma, and further liquid tumors, e.g. leukemias.

Intraocular neovascular syndromes are e.g. diabetic retinopathy, neovascular glaucoma, ischemic retinopathies and macular degeneration, e.g. age-related macular degeneration. Other angiogenesis related diseases are restenosis, e.g. stent-induced restenosis, Crohn's disease, and Hodgkin's disease.

Furthermore the invention relates to a method of treatment of angiogenesis dependent cancers and intraocular neovascular syndromes in a patient in need thereof, characterized in that a therapeutically effective amount of a compound of formula 1 as described hereinbefore as such or in form of a pharmaceutical preparation comprising it is administered to the patient in need thereof.

Another aspect of the invention relates to a compound according to any of the claims 1 to 3 or a pharmaceutical preparation according to claim 4 for use in a method for treatment of cancer or intraocular neovascular syndromes, in particular for a method for treatment of cancer. The compounds of the general formula 1 can be administered as such or especially in the form of pharmaceutical preparations, preferably in an amount effective against the said diseases, to a warm-blooded animal, for example a human, requiring such treatment. In the case of an individual having a bodyweight of about 70 kg the daily dose administered is from approximately 0.05 g to approximately 5 g, preferably from approximately 0.25 g to approximately 1.5 g, of a compound of the present invention.

### Short description of the Figures:

Fig.1 shows the selectivity profile of compound 2 with respect to protein kinases;
Fig.2 shows the selectivity profile of dasatinib with respect to protein kinases as published by DiscoveRx at www.discoverx.com/tools-resources/interaction-maps; the legend applicable to Fig. 2-4 is in Fig. 2;
Fig.3 shows the selectivity profile of erlotinib with respect to protein kinases as published by DiscoveRx at www.discoverx.com/tools-resources/interaction-maps;
Fig.4 shows the selectivity profile of sunitinib with respect to protein kinases as published by DiscoveRx at www.discoverx.com/tools-resources/interaction-maps;
Fig.5 shows the log base-10 values of GI₅₀, TGI and LC₅₀ (as defined and measured according to the Examples section hereafter) of compound 2, as well as a graphical representation of the Z-score of each given value (GI₅₀, TGI, LC₅₀) for a given cell line relative to the mean and standard deviation of that value for all 60 cell lines of the NCI-60 panel of cancer cell-lines
Fig.6 shows the effect of compound 2 on MDA-MB-231 breast cancer cells implanted subcutaneously in xenograft mouse models.

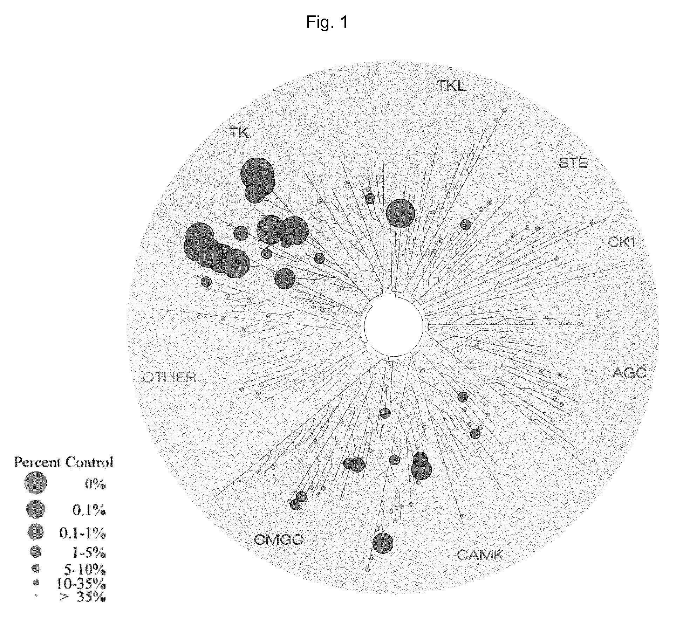

A selectivity profile of compound 2 with respect to protein kinases is depicted in Figure 1. The protein kinases are selected from tyrosine kinases (TK), tyrosine kinase-like kinases (TKL), STE-Group, Cell Kinases 1 (CK1), AGC-Group, Calmodulin/Calcium regulated kinases (CAMK), CMGC Group and other kinases. The larger the respective dot, the higher the binding affinity of the compound to the respective protein kinases. Compound 2 shows selectivity towards tyrosine kinases (TK). Furthermore compound 2 shows a selective binding affinity relative to one or more of the following protein kinases: ABL1(F317L)-phosphorylated, ABL1 (H396P)-phosphorylated, ABL1 (Y253F)-phosphorylated, ABL1-phosphorylated, ABL2, ACVR2B, BLK, BMPR1 B, BRAF(V600E), BRK, CSF1R, CSK, DDR1, EPHA1, EPHA2, EPHA4, EPHA5, EPHA8, EPHB1, EPHB2, EPHB3, EPHB4, EPHB6, FGR, FLT3, FLT3(ITD), FRK, FYN, HCK, KIT, KIT(D816V), KIT(V559D), KIT(V559D, V654A), LCK, LYN, PDGFRB, PKMYT1, RET, RET(M918T), SRC, TGFBR1, TNNI3K, TXK, YES. As per Kawasaki Y. and Freire E. in Drug Discovery Today, 2011 November; 16(21-22): 985-990, "[e]xtremely high affinity and selectivity are two of the most sought-after properties of drug molecules."

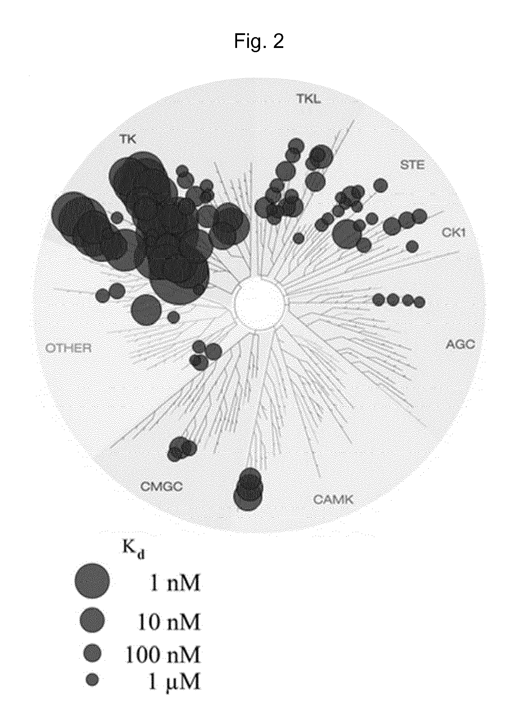

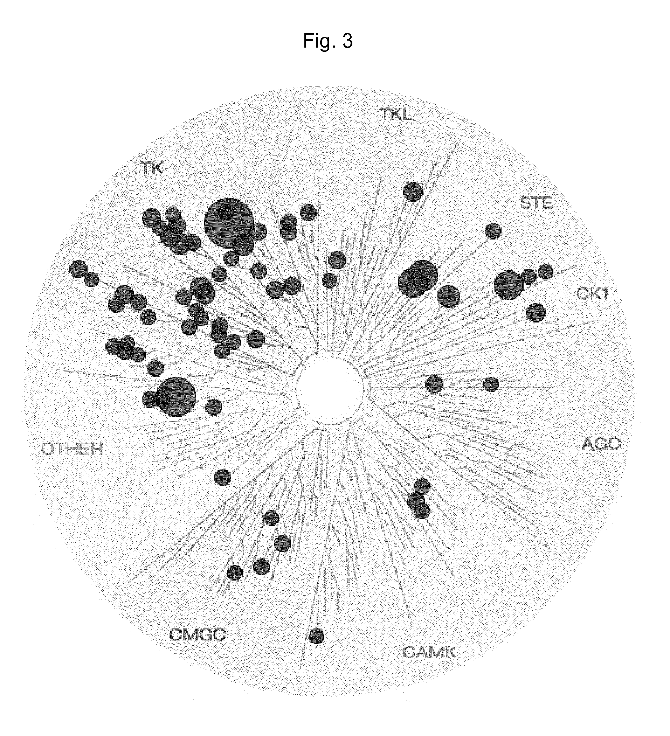

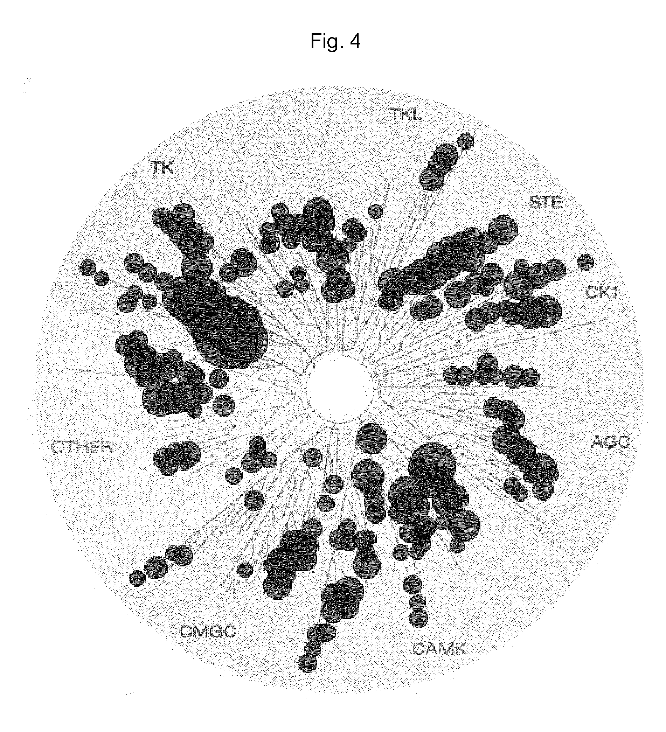

Figure 2 (dasatinib; *N*-(2-chloro-6-methylphenyl)-2-[[6-[4-(2-hydroxyethyl)-1-piperazinyl]-2-methyl-4-pyrimidinyl]amino]-5-thiazole carboxamide), Figure 3 (erlotinib; N-(3-Ethinylphenyl)-6,7-bis- (2-methoxyethoxy)chinazolin-4-amine) and Figure 4 (sunitinib; -[2-(Diethylamino)ethyl]-5-[(Z)- (5-fluor-1,2-dihydro-2-oxo-3H-indol-3-yliden)- methyl] -2,4-dimethyl-1 H-pyrrol-3-carboxamide) show the selectivity of FDA approved known compounds for comparison.

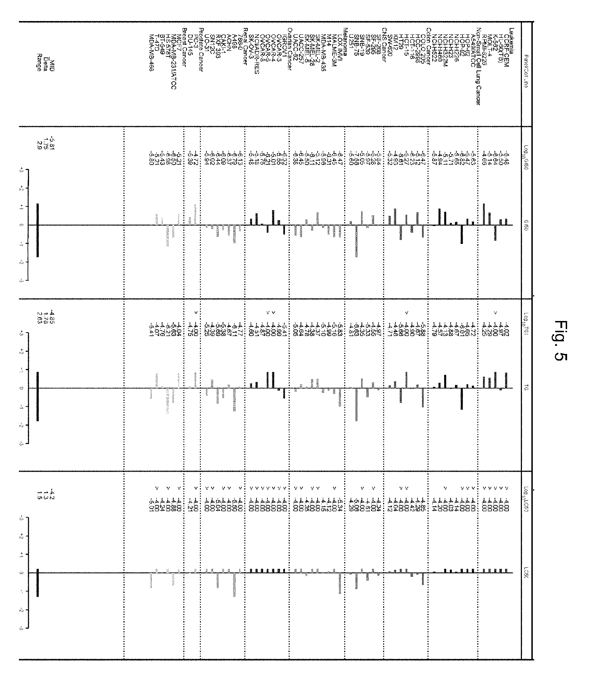

Compound 2 was screened by the National Cancer Institute (NCI) at 5 doses on the NCI-60 DTP Human Tumor Cell Line Screen. Figure 5 shows the high potency (low GI50 and/or TGI and/or LC50) of compound 2 relative to select cell-lines, including but not limited to the SNB-75 glioblastoma-grade IV cells, K562 myelogenous leukemia cells, A498 renal carcinoma cells, LOX IMV1 melanoma cells, and HS 578T and MDA-MB-231 breast cancer cells.

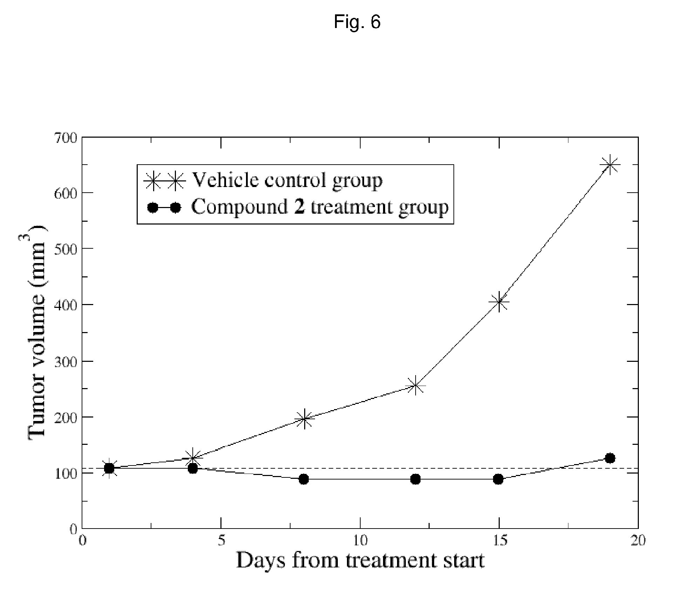

The effect of compound 2 on MDA-MB-231 breast cancer cells was examined. The breast cancer cells were implanted subcutaneously in xenograft mouse models and the development of the body weight and the tumor volume were observed over time (21 days). Group 1 is the vehicle control group consisting of 9 animals, whereas group 2 consisted of 9 animals receiving 50 mg/kg of compound 2 twice-daily. As could be seen in Figure 6, compound 2 provides a remarkable group-median tumor volume control.

### Examples

### General methods:

All reactions, unless otherwise stated, were carried out under a nitrogen atmosphere using standard Schlenk-techniques. All reagents were used as received unless otherwise noted. Solvents were purchased in the best quality available, degassed by purging thoroughly with nitrogen and dried over activated molecular sieves of appropriate size.

Alternatively, they were purged with argon and passed through alumina columns in a solvent purification system (Innovative Technology). Reactions were monitored by thin layer chromatography (TLC) using Merck TLC silica gel 60 F₂₅₄. Flash column chromatography was performed over silica gel (230-400 mesh). NMR spectra were recorded on AV2 400 or AV2 500 MHz Bruker spectrometers. Chemical shifts are given in ppm. The spectra are calibrated to the residual ¹H and ¹³C signals of the solvents.

Multiplicities are abbreviated as follows: singlet (s), doublet (d), triplet (t), quartet (q), doublet-doublet (dd), quintet (quint), septet (sept), multiplet (m), and broad (br). Melting points were determined on a Büchi Melting Point B-540 instrument. 2,3-Dichloroquinoxaline, o-toluidine, 5-methoxy-2-methylaniline, 3-amino-4-methylbenzyl alcohol, 2,6-dimethylaniline, 2-chloro-6-methylaniline were purchased from Fluka.

High-resolution electrospray ionization mass spectrometry was performed on a Finnigan MAT 900 (Thermo Finnigan, San Jose, CA, USA) double-focusing magnetic sector mass spectrometer. Ten spectra were acquired. A mass accuracy ≤2 ppm was obtained in the peak matching acquisition mode by using a solution containing 2 µL PEG200, 2 µL PPG450, and 1.5 mg NaOAc (all obtained from Sigma-Aldrich, Buchs, Switzerland) dissolved in 100 mL MeOH (HPLC Supra grade, Scharlau, E-Barcelona) as internal standard.

### General synthesis of compounds of the invention:

A reaction pathway to the intermediate 5a is depicted in scheme 1.

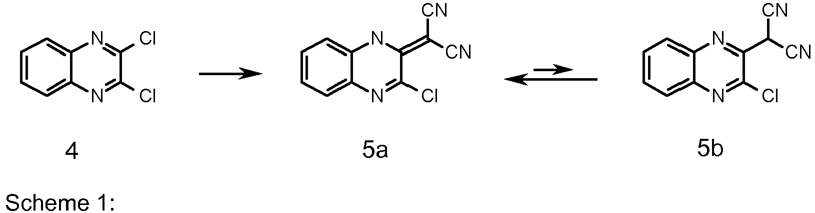

The following is taken from (a) Pratt, E.F. and Kereszte.Jc. "Syntheses of Indolizino- and Dihydroindolizinoquinoxalines"J Org Chem 1967, 32 (1):49-53, and (b) Obafemi, C.A.; Pfleiderer, W. "Synthesis and some reactions of 3-chloro-2-(cyanomethylene)-1,2-dihydroquinoxalines." Molecules 2004, 9 (4):223-231. To a solution of 10.45 mmol NaH in anhydrous dimethoxyethane, 10.04 mmol malononitrile was added. The reaction mixture was stirred at 25 °C for 30 min and 5.02 mmol 2,3-dichloroquinoxaline (4) was added. The resulting mixture was stirred at 25 °C for 3 h and then heated to reflux for one additional hour. The reaction mixture was cooled to 0 °C and 30 mL of 0.4 M HCl was slowly added. After stirring for 30 min at 0 °C, the formed precipitate was filtered off and recrystallized in EtOH to afford 2-(3-Chloroquinoxalin-2(1 H)-ylidene)malononitrile (5a) in 74% yield.

A reaction pathway to the general formula 1 is depicted in scheme 2.

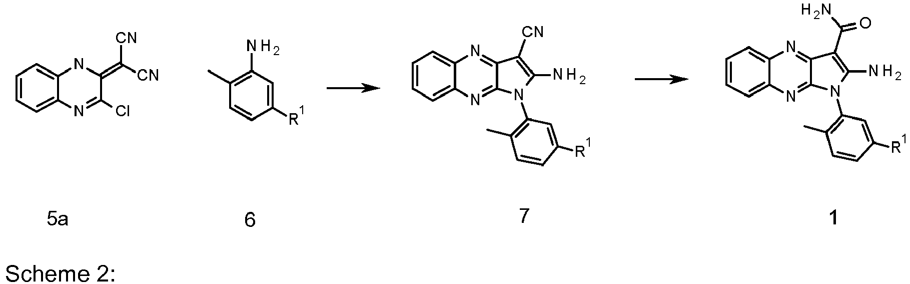

To a mixture of 1 eq 2-(3-chloroquinoxalin-2-yl)malononitrile (5a) in a reaction solvent 4 eq of the primary amine (6) was added. The reaction mixture was heated to a reaction temperature in the range of 50 to 150 °C for 2 to 4 h, in particular to 130 °C for 3 h, cooled, and the formed solid was filtered off and washed with a washing solution to afford the corresponding carbonitrile (7) in pure form.

In some embodiments, the reaction solvent is selected from the group consisting of toluene, methanol, ethanol (EtOH), isopropanol, dimethylformamide and mixtures thereof, and is preferably EtOH, toluene or a mixture thereof. In some embodiments, the reaction solvent is a 1:1 EtOH:Toluene solution. In some embodiments, the reaction mixture is heated to a reaction temperature of 130 to 160 °C for 3 h. In some embodiments, the washing solution is selected from the group consisting of EtOH, ether, hexane, pentane, toluene or mixtures thereof, and is preferably EtOH. In some embodiments, the reaction product is concentrated under reduced pressure and purified by flash chromatography on silica gel.

Subsequently 1 eq of the carbonitrile (7) was dissolved in 0.15 M sulfuric acid and poured in ice cold basified water and the formed solid was filtered off and optionally washed with a washing solution. Extraction afforded the corresponding products (1) in pure form, wherein optionally the extraction is carried out at a certain temperature (e.g. 30 to 50 °C, preferably around 25°C). Optionally, the desired product may be washed with a washing solution. The organic layer was dried over MgSO₄, filtered, and concentrated under reduced pressure yields the product. Optional purification by column chromatography on silica gel may be applied. Additionally or alternatively the product may be recrystallized.

In some embodiments, the basified water has a pH in the range of 6.7 to 7.3, and in particular of 7.0. In some embodiments, the water was basified with a solution of ammonium hydroxide (25%), nitric oxide, NaOH or KOH. In some embodiments, the reaction product is extracted with EtOAc and a saturated solution of NaHCO₃, EtOAc, acetone. In some embodiments, the reaction product is extracted at a temperature in the range of 20 to 30 °C. In some embodiments, the reaction product is extracted at a temperature of 160 °C with acetone. In some embodiments, the washing solution is selected from the group of ethanol, hexane and water. In some embodiments, the washing solution is water and hexane. In some embodiments, solvents for recrystallization are selected from the group consisting of ethanol and acetone. In some embodiments, a solvent for recrystallization is acetone.

The primary amine is 3-amino-4-methyl-phenol (6a) for the production of compound 2 or 3-amino-4-methyl-phenyl)methanol (3-Amino-4-methylbenzyl alcohol) (6b) for the production of compound 3, if a reaction pathway according scheme 1 and 2 is applied.

Alternatively, compound 2 can be produced by a selective reduction of the respective methoxy-compound (8). The reaction pathway is depicted in scheme 3:

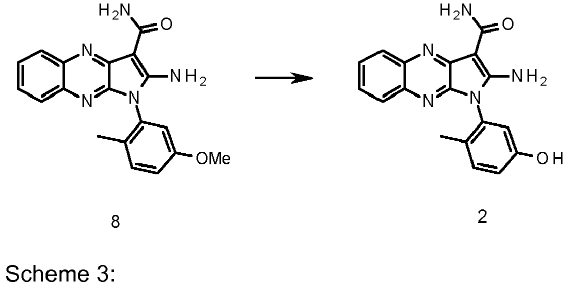

Compound 8 can be produced according to the reaction pathway depicted in scheme 1 and 2 by using 5-methoxy-2-methyl-aniline as the primary ammine (6c). A selective reduction to compound 2 may be achieved by using for example BBr₃ in anhydrous CH₂Cl₂ at -78 °C and refluxing.

### Synthesis and Characterization

### 2-Amino-1-(5-hydroxy-2-methylphenyl)-1H-pyrrolo[2,3-b]quinoxaline-3-carboxamide (2)

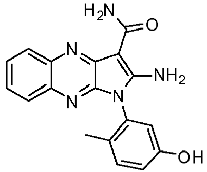

To a solution of 2-amino-1-(5-methoxy-2-methylphenyl)-1 H-pyrrolo[2,3-b]quinoxaline-3-carboxamide (8, 135 mg, 0.39 mmol) in anhydrous CH₂Cl₂ (5 mL) at -78 °C was added BBr₃ (1 M in CH₂Cl₂, 972 µL, 0.97 mmol). The reaction was allowed to warm to room temperature and was then heated for 1 h to 130 °C. The formed solid was filtered off and purified by flash chromatography on silica gel (gradient EtOAc:MeOH 99:1 to 98:2) to afford the desired product in pure form (48 mg, 37% yield).

¹H NMR (500 MHz, DMSO-*d₆*): δ = 9.73 (s, 1 H), 8.02 (s, 2H), 7.94 (dd, *J* = 8.3 Hz, *J* = 1.3 Hz, 1 H), 7.78 (dd, *J* = 8.2 Hz, *J* = 1.3 Hz, 1 H), 7.75 (s, 1 H), 7.57 (ddd, *J* = 8.3 Hz, *J* = 6.9 Hz, *J* = 1.3 Hz, 1 H), 7.46 (ddd, *J* = 8.2 Hz, *J* = 6.9 Hz, *J* = 1.3 Hz, 1 H), 7.37 (s, 1 H), 7.30 (d, *J* = 8.4 Hz, 1 H), 6.94 (dd, *J* = 8.4 Hz, *J* = 2.5 Hz, 1 H), 6.81 (d, *J* = 2.5 Hz, 1 H), 1.88 (s, 3H);
¹³C NMR (125 MHz, DMSO-*d₆*): *δ* = 166.6, 158.8, 156.3, 143.0, 141.4, 139.6, 136.8, 131.9, 131.3, 127.6, 126.8, 126.6, 125.1, 117.2, 116.2, 82.4, 16.2, one C missing due to overlapping;
IR (film): *υ̃* = 3455, 3380, 3292, 3200, 1633, 1511, 1461, 1021, 753, 595 cm⁻¹;
HRMS (ESI): m/z: calcd for C₁₈H₁₅N₅O₂H⁺: 334.1299, found: 334.1296.

### 2-Amino-1-(5-(hydroxymethyl)-2-methylphenyl)-1H-pyrrolo[2,3-b]quinoxaline-3-carboxamide (3)

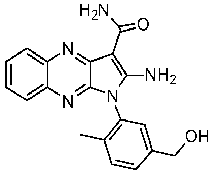

A solution of 2-amino-1-(5-(hydroxymethyl)-2-methylphenyl)-1H-pyrrolo[2,3-b]quinoxaline-3-carbonitrile (7b, 186 mg, 0.56 mmol) in sulfuric acid (3 mL) was stirred for 30 min at 25 °C. The reaction was poured in ice cold water and the formed solid was filtered off and washed with water and hexane. The crude product was then heated in acetone (5 mL) at 160 °C for 1 h. After cooling to room temperature, the solid was filtered off and washed with hexane to afford the desired product in pure form (143 mg, 72% yield).

¹H NMR (500 MHz, DMSO-*d₆*): δ = 8.03 (s, 2H), 7.94 (dd, *J* = 8.3 Hz, *J* = 1.3 Hz, 1 H), 7.77-7.75 (m, 2H), 7.57 (ddd, *J* = 8.3 Hz, *J* = 7.0 Hz, *J* = 1.3 Hz, 1 H), 7.49-7.48 (m, 2H), 7.46 (ddd, *J* = 8.3 Hz, *J* = 7.0 Hz, *J* = 1.3 Hz, 1 H), 7.39-7.36 (m, 2H), 5.34 (t, *J* = 5.6 Hz, 1 H), 4.57 (d, *J* = 5.6 Hz, 2H), 2.00 (s, 3H);

¹³C NMR (125 MHz, DMSO-*d₆*): *δ* = 166.6, 159.0, 143.1, 142.0, 141.4, 139.6, 136.8, 135.3, 131.0, 130.7, 127.9, 127.6, 127.4, 126.8, 126.6, 125.1, 82.5, 62.0, 16.9;
IR (film): *υ̃*= 3339, 3247, 3162, 1661, 1603, 1148, 1036, 876, 762, 702 cm⁻¹;
HRMS (ESI): m/z: calcd for C₁₉H₁₇N₅O₂H⁺: 348.1455, found: 348.1458.

### 2-(3-Chloroquinoxalin-2(1H)-ylidene)malononitrile (5a)

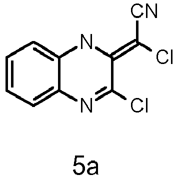

The following is taken from (a) Pratt, E. F. and Kereszte.Jc. "Syntheses of Indolizino- and Dihydroindolizinoquinoxalines." J Org Chem 1967, 32 (1):49-53, and (b) Obafemi, C. A.; Pfleiderer, W. "Synthesis and some reactions of 3-chloro-2-(cyanomethylene)-1,2-dihydroquinoxalines." Molecules 2004, 9 (4):223-231. To a solution of NaH (250 mg, 10.45 mmol) in anhydrous dimethoxyethane (20 mL) was carrefully added malononitrile (663 mg, 10.04 mmol). The reaction mixture was stirred at 25 °C for 30 min and 2,3-dichloroquinoxaline (4, 1 g, 5.02 mmol) was added. The resulting mixture was stirred at 25 °C for 3 h and then heated to reflux for one additional hour. The reaction mixture was cooled to 0 °C and 0.4 M HCl (30 mL) was slowly added. After stirring for 30 min at 0 °C, the formed precipitate was filtered off and recrystallized in EtOH to afford compound 5a in pure form (845 mg, 74% yield).

¹H NMR (400 MHz, DMSO-*d₆*): δ = 7.67-7.62 (m, 2H), 7.57 (ddd, *J* = 8.4 Hz, *J* = 6.8 Hz, *J* = 1.1 Hz, 1 H), 7.36 (ddd, *J* = 8.1 Hz, *J* = 6.8 Hz, *J* = 1.2 Hz, 1 H), NH-not observed;
¹³C NMR (100 MHz, DMSO-*d₆*): *δ* = 149.3, 141.9, 134.8, 134.1, 131.3, 127.5, 125.5, 120.2, 118.6, 44.5, one C missing due to overlapping;
IR (film): *υ̃*= 3235, 3203, 3100, 3056, 3013, 2979, 2220, 2208, 1616, 1577, 1488, 1410, 1096, 969, 799, 764, 594 cm⁻¹;
MS (ESI): m/z: calcd for C₁₁H₅ClN₄Na⁺: 251.0, found: 250.9.

### 2-Amino-1-(5-(hydroxymethyl)-2-methylphenyl)-1H-pyrrolo[2,3-b]quinoxaline-3-carbonitrile (7b)

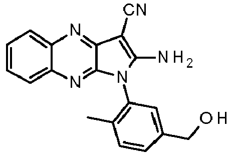

To a mixture of 2-(3-chloroquinoxalin-2-yl)malononitrile (5a, 1 eq) in a 1 : 1 EtOH-Toluene solution (0.09 M), the primary amine (6b, 4 eq of (3-amino-4-methylphenyl)methanol; HOCH₂C₆H₃(CH₃)NH₂) was added. The reaction mixture was heated to 130 °C for 3 h, cooled, and the formed solid was filtered off and washed with EtOH to afford the corresponding products in pure form.
Yield: 74%;

¹H NMR (500 MHz, DMSO-*d₆*): δ = 8.28 (s, 2H), 7.94 (d, *J* = 8.0 Hz, 1 H), 7.75 (d, *J* = 7.8 Hz, 1 H), 7.58 (ddd, *J =* 8.0 Hz, *J =* 7.1 Hz, *J =* 1.0 Hz, 1 H), 7.49-7.47 (m, 3H), 7.34 (s, 1 H), 5.33 (t, *J* = 5.5 Hz, 1 H), 4.56 (d, *J* = 5.5 Hz, 2H), 1.98 (s, 3H);

¹³C NMR (125 MHz, DMSO-*d₆*): *δ* = 159.7, 143.6, 142.3, 142.1, 140.3, 137.2, 135.3, 131.1, 130.5, 128.1, 127.5, 127.4, 127.1, 126.8, 125.7, 115.1, 62.0, 60.3, 16.8;
IR (film): *υ̃*= 3343, 3309, 3124, 2208, 1637, 1550, 1446, 766, 599 cm⁻¹;
HRMS (ESI): *m*/*z:* calcd for C₁₉H₁₅N₅OH⁺: 330.1349, found: 330.1346.

2-Amino-1-(5-methoxy-2-methylphenyl)-1H-pyrrolo[2,3-*b*]quinoxaline-3-carbonitrile (7c)

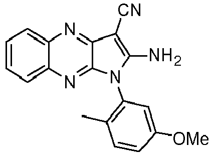

To a mixture of 2-(3-chloroquinoxalin-2-yl)malononitrile (5a, 1 eq) in a 1 : 1 EtOH-Toluene solution (0.09 M), the primary amine (6c, 4 eq of 5-methoxy-2-methyl-aniline; CH₃OC₆H₃(CH₃)NH₂) was added. The reaction mixture was heated to 130 °C for 3 h, cooled, and the formed solid was filtered off and washed with EtOH to afford the corresponding products in pure form (Yield: 53%).

¹H NMR (500 MHz, DMSO-*d₆*): δ = 8.30 (s, 2H), 7.94 (dd, *J* = 8.3 Hz, *J* = 1.3 Hz, 1 H), 7.77 (dd, *J* = 8.3 Hz, *J* = 1.3 Hz, 1 H), 7.58 (ddd, *J* = 8.3 Hz, *J* = 6.9 Hz, *J* = 1.3 Hz, 1 H), 7.48 (ddd, *J* = 8.3 Hz, *J* = 6.9 Hz, *J* = 1.3 Hz, 1 H), 7.41 (d, *J* = 8.5 Hz, 1 H), 7.13-7.09 (m, 2H), 3.77 (s, 3H), 1.91 (s, 3H);
¹³C NMR (125 MHz, DMSO-*d₆*): *δ* = 159.6, 158.3, 143.6, 142.2, 140.3, 137.3, 131.9, 131.4, 128.7, 127.5, 127.0, 126.8, 125.6, 116.2, 115.2, 115.1, 60.2, 55.4, 16.2;
IR (film): *υ̃* = 3315, 3144, 3061, 2208, 1636, 1546, 1506, 1447, 1313, 1209, 1030, 820, 760, 735 cm⁻¹;
HRMS (ESI): *m*/*z:* calcd for C₁₉H₁₅N₅OH⁺: 330.1349, found: 330.1348.

### 2-Amino-1-(5-methoxy-2-methylphenyl)-1H-pyrrolo[2,3-b]quinoxaline-3-carboxamide (8)

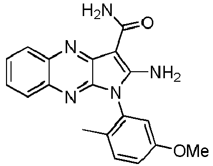

A solution of 2-amino-1-(5-(hydroxymethyl)-2-methylphenyl)-1H-pyrrolo[2,3-b]quinoxaline-3-carbonitrile (7c, 151.8 mg, 0.459 mmol) in sulfuric acid (3 mL) was stirred for 30 min at 25 °C. The reaction was poured in ice cold water, basified with a solution of ammonium hydroxide (25%) and the formed solid was filtered off. Extraction with EtOAc and a saturated solution of NaHCO₃ afforded the corresponding products in pure form, (115 mg, 72% yield).

¹H NMR (300 MHz, DMSO-*d₆*): *δ* = 7.94 (d, *J =* 7.7 Hz, 1 H), 7.79-7.76 (m, 2H), 7.57 (ddd, *J =* 8.2 Hz, *J =* 6.9 Hz, *J =* 1.3 Hz, 1 H), 7.49-7.37 (m, 3H), 7.15-7.09 (m, 2H), 3.78 (s, 3H), 1.94 (s, 3H), NH₂-not observed;
¹³C NMR (125 MHz, DMSO-*d₆*): *δ* = 166.6, 158.9, 158.2, 143.1, 141.5, 139.6, 136.8, 131.8, 131.6, 128.7, 127.6, 126.8, 126.6, 125.1, 115.9, 115.0, 82.5, 55.4, 16.3;
IR (film): *υ̃* = 3482, 3405, 3295, 3067, 2998, 2930, 1630, 1612, 1507, 1465, 1308, 1227, 1119, 1027, 817, 745, 596 cm⁻¹;
HRMS (ESI): *m*/*z:* calcd for C₁₉H₁₇N₅O₂H⁺: 348.1455, found: 348.1454.

### In vitro evaluation:

In the following, compounds 17 and 87 correspond to prior art compounds 1 and 2 of Figure 5 in Zhao H. and Huang D., "Hydrogen Bonding Penalty upon Ligand Binding", PLoS ONE, 2011, Volume 6, Issue 6. These were purchased from InterBioScreen.

### FRET based enzymatic assay:

Selected compounds were tested in the Z'-LYTE™ Kinase Assay Kit-Tyr 1 Peptide (Invitrogen, USA) in a Corning 384 well microtiter plate. Fluorescence progress curves were measured upon excitation at 400 nm and emission at 445 and 520 nm. The assay contained a final concentration of EphB4 and ATP of 25 ng/µL and 125 µM (which is near its Kₘ), respectively, and was run at room temperature for 2 h. IC₅₀ Values (inhibitor concentration at which enzyme activity is reduced by 50%) are determined after carrying out assays at ten different concentrations between 20 µM and 10 pM. Compound 2 comprises an IC₅₀ value on EphB4 of 11 nmol/L. In Zhao H. and Huang D. "Hydrogen Bonding Penalty upon Ligand Binding", PLoS ONE, 2011, compounds 17 and 87 (corresponding to compounds 1 and 2 of Figure 5 therein) are reported to comprise IC₅₀ values on EphB4 of 380 and 300 nmol/L, respectively.

### Cellular phosphorylation assays:

The cellular phosphorylation experiments were performed at ProQinase GmbH. Mouse embryonic fibroblast cells, stably transfected to overexpress full-length human EphB4, were plated at 4×10⁴ cells/well in DMEM supplemented with 10% FCS in 48-well culture dishes. Medium was replaced by DMEM without FCS before test compounds prediluted in 100% DMSO were added (final DMSO concentration of 1 %). After incubation for 90 min at 37 °C, cells were stimulated for 2 h at 4 °C using murine ephrinB2-Fc at a final concentration of 2 µg/ml. Quantification of EphB4 phosphorylation was assessed in a 96-well plate via sandwich ELISA using a myc capture antibody and an antiphosphotyrosine detection antibody. Compounds were tested for their inhibitory impact on EphB4 cellular kinase activity as assessed by measurement of EphB4 autophosphorylation. Compound 2 comprises a cellular IC₅₀ value of less than 6 nmol/L. Compound 3 comprises an IC₅₀ of 160 nmol/L. Prior art compound 17 comprises an IC₅₀ of 640 nmol/L. Comparison compound 8 comprises an IC₅₀ of 720 nmol/L.

### Cell culture and GI₅₀ determination:

MDA-MB-231 (breast cancer) and KM12 (colon cancer) cells obtained from the UZH Cancer Institute, HOP-92 (lung cancer) cells purchased from the National Cancer Institute (NCl) and A498 (kidney cancer) cells obtained from Prof. Krek (Institute of Cell Biology, ETH Zurich) were cultured in Dulbecco's modified Eagle's medium (DMEM) supplemented with 10% (v/v) fetal bovine serum. K562 leukemia cells obtained from Dr. Silvio Hemmi (Institute of Molecular Life Sciences, UZH) were cultured using RPMI medium supplemented with 5% (v/v) fetal bovine serum. All the media was additionally supplemented with 100 units/mL of penicillin, 100µg/mL of streptomycin, 4.5 g/L glucose, 0.11g/L sodium pyrubate and 2mM glutamine and the cells were grown at 37 °C in 5% CO₂ atmosphere with 80% relative humidity.

MDA-MB-231, HT-29, KM12, HOP-92 and A498 cells were plated at 10,000 cells per well (100 µL per well) in 96-well culture dishes and allowed to incubate for 24 h. The old media was removed, the cells were washed with PBS (phosphate-buffered saline) and fresh media was added. A 5 mmol/L solution of inhibitor (in 100% DMSO) was serially diluted in the culture media (12 different concentrations were used) and allowed to incubate for 72 h (MDA-MB-231) or 48h (A498). Control cells were treated with the same DMSO concentrations. After the incubation period the media was once more removed and the cells were washed with PBS to then be incubated with fresh media containing 86 nmol/L resazurin. After 3 hours, the fluorescence was quantified using a fluorescence microplate reader (Biotek, FLx800™) at the respective excitation and emission wavelength of 560 and 590 nm. Resazurin becomes fluorescent under mitochondrial reduction and its emission directly correlates with the metabolic stability of the cells. The measured fluorescence values were corrected from the control samples containing DMSO and GI₅₀ values were determined by plotting the fraction of metabolically active cells against the log of the drug concentration. All measurements were performed at least in triplicate.

In the case of K-562 leukemia cells, these were seeded at a density of 20,000 cells per well in 100µL of RMPI media in a 96 well microtiter plates. After 24 hours, 12.5 µL of a 10 fold concentrated test compound or DMSO solution in RMPI media was added in every well. After 48 hour incubation, cell viability was assessed by measuring the ability of the cells to process resazurin. Resazurin was added to every well to obtain a final concentration of 86nmol/L, and after 3 hours, the fluorescence was quantified using a fluorescence microplate reader (Biotek, FLx800™) at the respective excitation and emission wavelength of 560 and 590 nm as described above. All measurements were performed at least in triplicate.

**Table 1a: GI₅₀ MDA-MB-231**

| **Compound no.** | **GI50 (µmol/l)** |
|---|---|
| Compound 2 | 2.64 |

**Table 1b: GI₅₀ K562**

| **Compound no.** | **GI50 (µmol/l)** |
|---|---|
| Compound 2 | 1.05 |
| Prior Art 17 | 35.63 |
| Prior Art 87 | 73.07 |

**Table 1c: GI₅₀ A498**

| **Compound no.** | **GI50 (µmol/l)** |
|---|---|
| Compound 2 | 5.88 |
| Compound 8 | 59.29 |
| Prior Art 17 | 49.96 |
| Prior Art 87 | 99.64 |

**Table 1d: GI₅₀ HT-29**

| **Compound no.** | **GI50 (µmol/l)** |
|---|---|
| Compound 2 | 4.59 |

**Table 1e: GI₅₀ KM12**

| **Compound no.** | **GI50 (µmol/l)** |
|---|---|
| Compound 2 | 1.55 |

**Table 1f: GI₅₀ HOP-92**

| **Compound no.** | **GI50 (µmol/l)** |
|---|---|
| Compound 2 | 0.49 |

### NCl DTP Human Tumor Cell Line Screen:

Compound 2 was further screened against cancer cell lines by the National Cancer Institute (NCI). This screen was performed as per the description provided at http://dtp.nci.nih.gov/branches/btb/ivclsp.html. "The NCI 60 panel cell lines were grown in RPMI 1640 medium supplemented with 5% (v/v) fetal bovine serum and 2 mM L-glutamine at 37° C, 5 % CO2, 95 % air and 100 % relative humidity. Cells were seeded at 5,000 - 40,000 cells/well depending on their doubling time. After 24 hours of incubation, DMSO stock solutions of the compounds (400X) were added at 5 different concentrations (in 4, 10 or ½ log serial dilutions). After 48 h cells were fixed with trichloroacetic acid (10% or 16% final concentration for adherent cells and suspension cells respectively) and incubated for 60 minutes at 4°C. After discarding the supernatant, plates were washed with water (5x) and air dried. Sulforhodamine B (SRB) solution (100 µl) at 0.4 % (w/v) in 1% acetic acid was added to each well and incubated for 10 minutes at 4°C, followed by washing with 1% acetic acid (5x) and air drying. The bound stain was solubilized with 10mM trizma base and determined by absorbance measurement on an automated plate reader at a wavelength of 515 nm. The percentage growth inhibition was calculated as followed, being Tz time zero measurements, C control growth measurements and Ti test growth experiments in the present of the drug.
[(Ti-Tz)/(C-Tz)] x 100 for concentrations for which Ti>/=Tz
[(Ti-Tz)/Tz] x 100 for concentrations for which Ti<Tz.

Three dose response parameters were calculated for each cell line. Growth inhibition of 50% (GI₅₀) is calculated from [(Ti-Tz)/(C-Tz)] x 100 = 50 and corresponds to the drug concentration required in order to reduce by 50% the net protein increase in comparison to the control experiments. Total growth inhibition (TGI) is calculated from Ti = Tz. The LC50 indicates the drug concentration needed to reduce by 50% the measured protein at the end of the drug treatment as compared to that of the beginning and is derived from [(Ti-Tz)/Tz] x 100 = -50."

**Table 2: GI₅₀ in a selection of cancer cell lines of the National Cancer Institute for compound 2**

| **Cell line** | **GI50 (µmol/l)** |
|---|---|
| Renal RXF393 | 0.361 |
| Renal A498 | 0.162 |
| CNS SNB75 | 0.028 |
| Colon HT29 | 0.244 |
| Leukemia K562 | 0.229 |
| Breast HS578T/MDA-MB-231 | 0.100/0.317 |
| NSCLC HOP92 | 0.152 |

### Surface Plasmon Resonance (SPR) determination of binding and unbinding kinetics:

The affinity of compound 2 towards EphA3 was determined using Surface Plasmon Resonance measurements on a ProteOn XPR36 (Bio-Rad Laboratories, AG). EphA3 was immobilized on a GLH chip (Bio-Rad Laboratories AG) by standard amine coupling procedures achieving a RU of 4000-6000 and equilibrated with steriled-filtered buffer (10mM HEPES, 150mM NaCl, 3mM EDTA, 0.005% Tween 20 pH 8.0) with a flow rate of 60µL/min. Association of compound 2 was performed at 25 °C using serial dilutions of the inhibitors in duplicates for 400s, whereas dissociation of the inhibitors from the chip was monitored for 36,000 seconds. Data were normalized using interspot referencing and subtraction of a separate analyte channel run with buffer and BSA. All sensograms were fitted using a 1:1 Langmuir model provided by the ProteOn Manager Software (Bio-Rad Laboratories AG), and the association (kₒₙ) and dissociation (k_{eff}) rate constants were used to determine the equilibrium dissociation constants (K_{D}).

Compound 2 comprises a dissociation constant (KD) of 6 nmol/L, an association rate constant kon of 2 * 106/µs (the association rate constant characterizes how quickly the compound binds to its target) and τoff of 658 s (is the residence time, the time the compound remains associated on the target; τoff is the reciprocal value of koff, which is the dissociation rate constant). According to the abstract by Manley P. et al. entitled "Nilotinib, in Comparison to Both Dasatinib and Imatinib, Possesses a Greatly Prolonged Residence Time When Bound to the BCR-ABL Kinase SH1 Domain" of the December 2011 53rd ASH (American Society of Hematology) Annual Meeting and Exposition (https://ash.confex.com/ash/2011/webprogram/Paper35985.html), the residence time is comparable with that of marketed drugs: imatinib (28 min for dephosphorylated ABL), nilotinib (202 or 205 min for dephosphorylated and phosphorylated ABL, respectively) and dasatinib (15 or 4 min). Long residence time favors not only in vivo drug efficacy but also in vivo selectivity due to the resulting high local concentration of the drug in the vicinity of the target (Nat. Rev. Drug Discov. 2006, 5, 730-739, J. Biomol. Screen September 30, 2013).

### Differential Scanning Fluorimetry:

To a 2 µmol/L solution of EphA3 in 10mM Tris HCl buffer (pH 8.0) containing 100mM NaCl, 5X SYPRO Orange fluorescent dye together with the test compounds prediluted in DMSO at a concentration of 20 µmol/L (1.4% final DMSO concentration). A final volume of 40 µL was added per well in a 96-well plate and incubated at room temperature for 1 h. The plates were centrifuged for 3 min at 450 rcf. The temperature gradient was performed in the range of 25 to 75 °C (50 acquisitions per degree at a ramp rate of 4.4 °C/s) using a LightCycler 480 Real-Time PCR System. The detection of protein unfolding was performed with an excitation wavelength of 488 nm and an emission of 580 nm. All measurements were performed in triplicate. Compound 2 comprises a difference in the melting temperature Tₘ (corresponding to the temperature at which the concentrations of folded and unfolded protein are equal) with respect to EphA3 of 11.44 °C (Δ Tₘ), whereas prior art compounds 17 and 87 presented differences in the melting temperature Tₘ with respect to EphA3 of 1.6 and 0.7 °C, respectively. The high shift in melting temperature provided by compound 2 attests to it strongly binding EphA3, thereby significantly shifting the protein melting temperature.

### In vivo evaluation:

### Pharmacokinetics:

Evaluation of the in vivo pharmacokinetics of a subset of compounds was performed through analysis of compound concentration in the blood plasma of male CD-1 (ICD) mice (each weighing 20-30 g) over a 24 hour period (at time points of 3, 10, 30, 60, 120, 240, 360 and 1440 minutes) following a single oral (PO) and intravenous (IV, in tail vein) administration of a compound dose corresponding to 5 mg of compound/kg of mouse body weight (PO) and 1 mg of compound/kg of mouse body weight (IV). For IV dosing, each compound was formulated as a clear solution in DMSO/Solutol® HS 15/phosphate-buffered saline, pH 7.4 (PBS) (5/5/90, v/v/v). For PO dosing, compounds were formulated as a homogeneous suspension in DMSO/1% methylcellulose (5/95, v/v).

At the relevant time point, blood samples (300-400 µL) were collected from the mice under general inhalant anaesthesia by cardiac puncture and stored on ice in 0.6 mL polypropylene heparin-lithium-coated tubes. Within 1 hour of collection, each tube was centrifuged at 2500 G for 15 minutes at 4°C and harvested plasma stored at -20°C.

Compound concentration in plasma at each time point was determined following acetonitrile precipitation of each sample followed by HPLC-MS analysis performed in triplicates, i.e., based on the mean of the compound concentration at that time point in blood samples of three mice.

In the following table 3:
T1/2 is the terminal elimination half-life following IV and PO dosing (obtained from non-compartmental analysis (NCA) of the plasma data using WinNonLin).
Tmax is the time following PO administration at which the maximum concentration is observed (obtained from non-compartmental analysis (NCA) of the plasma data using WinNonLin).
Cmax corresponds to the concentration observed at Tmax following PO administration (obtained from non-compartmental analysis (NCA) of the plasma data using WinNonLin).
Bioavailability was calculated as the ratio of:
   [AUClast(PO)*Dose(IV)] / [AUClast(IV)*Dose(PO)]
   where AUClast is the area under the curve (estimated by summing incremental areas of a series of trapezoids) of a plot of compound concentration versus time, up to the time of the last observation that is greater than the limit of quantification.

**Table 3: In vivo pharmacokinetics (min: minutes)**

| **Compound no.** | **T1/2 (min, IV)** | **T1/2 (min, PO)** | **Cmax (ng/ml, PO)** | **Tmax (min, PO)** | **Bioavailibility (%)** |
|---|---|---|---|---|---|
| Compound 2 | 101 | 100 | 240 | 30 | 25 |

### Efficacy in a Xenograft Mouse Model:

9 week old female NCr Nu/Nu mice (Charles River Laboratories, Inc.) were injected at the flank with 5x10⁶ MDA-MB-231 cells. Once tumor volume reached 108-126 mm³ (as measured by digital caliper) mice were randomized into a treatment and vehicle-control group of 9 animals each. The treatment group received oral doses of 50 mg/kg of compound 2 twice daily for 21 days (with the exception of a single dose on the first day), formulated as a homogeneous suspension in DMSO/1% methylcellulose (5/95, v/v). The control group received the same formulation but excluding compound 2. Tumor volume of all mice was evaluated bi-weekly up to day 19. Median tumor volume progression of both groups is given in Figure 6.

## Claims

1. A compound **characterized by** a general formula (1), 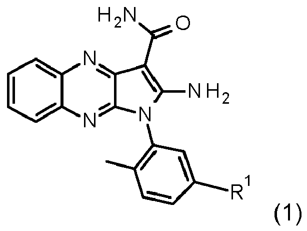 wherein R¹ is OH or CH₂OH.

2. The compound according to claim 1, wherein R¹ is OH.

3. The compound according to any of the previous claims, wherein the compound is isolated in its free form, in form of a salt, a tautomer, a pharmaceutically acceptable salt, a hydrate or a solvate

4. A pharmaceutical preparation comprising at least one compound according to any one of the claims 1 to 3 as an active ingredient.

5. A compound according to any of the claims 1 to 3 or a pharmaceutical preparation according to claim 4 for use in a method of treatment of disease.

6. A compound according to any of the claims 1 to 3 or a pharmaceutical preparation according to claim 4 for use in a method of treatment of cancer or intraocular neovascular syndromes, in particular for use in a method of treatment of cancer.

7. A dosage form for use in a method for preventing of treating of disease, in particular in a method for treatment of cancer or intraocular neovascular syndromes, comprising a compound according to any one of claims 1 to 3.

8. A method for manufacture a medicament for use in a method for preventing or treating of disease, in particular in a method for treatment of cancer or intraocular neovascular syndromes, comprising the use of a compound according to any one of claims 1 to 3.

9. A method for preventing or treating of disease, in particular a method for preventing or treating of cancer or intraocular neovascular syndromes, comprising the administration of a compound according to any one of claims 1 to 3 or a pharmaceutical preparation according to claim 4 to a patient in need thereof.
